# EUROPEAN PATENT SPECIFICATION

(11) **EP 1 455 813 B1**
(45) Date of publication and mention of the grant of the patent: **15.07.2015**
(21) Application number: 02782602.3
(22) Date of filing: 12.12.2002
(51) Int. Cl.: A61K 38/21, C12Q 1/68, C12N 15/10, A61P 11/00

(54) **INTERFERON GAMMA IN COMBINATION WITH A DIAGNOSTIC ARRAY FOR USE IN THE IMPROVED TREATMENT OF IDIOPATHIC PULMONARY FIBROSIS**
GAMMA-INTERFERON ZUR VERWENDUNG IN DER KOMBINATION MIT EINEM DIAGNOSICHEN CHIP IN DER VERBESSERTEN BEHANDLUNG VON IDIOPATHISCHER PULMONARER FIBROSE
INTERFERON GAMMA DANS COMBINAISON AVEC UNE PUCE DE DIAGNOSTIC MEDICAL POUR UNE UTILISATION DANS LE TRAITEMENT AMÉLIORÉ LA FIBROSE PULMONAIRE IDIOPATHIQUE

(30) Priority: 18.12.2001 EP 01130011
(43) Date of publication of application: 15.09.2004
(73) Proprietor: mondoBIOTECH AG, 6371 Stans (CH)
(72) Inventor: BEVEC, Dorian, CH-6925 Gentilino (CH); ZIESCHE, Rolf, A-1210 Wien (AT)
(86) International application number: PCT/CH2002/000691
(87) International publication number: WO 2003/051388

(56) References cited:
- EP-A- 0 316 170
- EP-A- 0 789 081
- EP-A- 0 795 332
- WO-A-00/17391
- WO-A-00/60117
- WO-A-01/34180
- WO-A-01/36001
- WO-A-94/26249
- AFFYMETRIX: "GeneChip Human Genome U133 Set" INTERNET ARTICLE, [Online] XP002232760 Retrieved from the Internet: <URL:http://www.affymetrix.com/support/tec hnical/datasheets/hgu133_datasheet.pdf> [retrieved on 2003-02-26]
- AFFYMETRIX: "GeneChip E.coli antisense genome array" INTERNET ARTICLE, [Online] XP002232761 Retrieved from the Internet: <URL:http://www.affymetrix.com/support/tec hnical/datasheets/ecoli_antisense_datashee t.pdf> [retrieved on 2003-02-26]
- AFFYMETRIX: "GeneChip Pseudomonas aeruginosa genome array" INTERNET ARTICLE, [Online] XP002232762 Retrieved from the Internet: <URL:http://www.affymetrix.com/support/tec hnical/datasheets/pseudomonas_datasheet.pd f> [retrieved on 2003-02-26]
- AFFYMETRIX: "GenGhip Yeast genome S98 array" INTERNET ARTICLE, [Online] XP002232763 Retrieved from the Internet: <URL:http://www.affymetrix.com/support/tec hnical/datasheets/yeast_datasheet.pdf> [retrieved on 2003-02-26]
- ZIESCHE ROLF ET AL: "A preliminary study of long-term treatment with interferon gamma-1b and low-dose prednisolone in patients with idiopathic pulmonary fibrosis." NEW ENGLAND JOURNAL OF MEDICINE, vol. 341, no. 17, 21 October 1999 (1999-10-21), pages 1264-1269, XP001145933 ISSN: 0028-4793
- ZIESCHE ROLF ET AL: "Mechanisms of antifibrotic action of interferon gamma-1b in pulmonary fibrosis." WIENER KLINISCHE WOCHENSCHRIFT, vol. 112, no. 18, 2000, pages 785-790, XP009005957 ISSN: 0043-5325
- ZIESCHE R ET AL: "[New therapeutic approaches in pulmonary fibrosis]" PNEUMOLOGIE (STUTTGART, GERMANY) GERMANY OCT 2000, vol. 54, no. 10, October 2000 (2000-10), pages 431-433, XP009005956 ISSN: 0934-8387
- KITA Y ET AL: "CHARACTERIZATION OF A POLYETHYLENE GLYCOL CONJUGATE OF RECOMBINANT HUMAN INTERFERON-GAMMA" DRUG DESIGN AND DELIVERY, HARWOOD ACADEMIC PUBLISHERS GMBH, XX, vol. 6, no. 3, 1990, pages 157-167, XP001042224
- RAGHU G ET AL: "TREATMENT OF IDIOPATHIC PULMONARY FIBROSIS WITH A NEW ANTIFIBROTIC AGENT, PIRFENIDONE. RESULTS OF A PROSPECTIVE, OPEN-LABEL PHASE II STUDY" AMERICAN JOURNAL OF RESPIRATORY AND CRITICAL CARE MEDICINE, AMERICAN LUNG ASSOCIATION, NEW YORK, NY, US, vol. 159, no. 4, April 1999 (1999-04), pages 1061-1069, XP000878900 ISSN: 1073-449X
- MARGOLIN S ET AL: "REMOVAL OF INTERSTITIAL PULMONARY FIBROSIS (ASBESTOS-INDUCED) BY ORAL CHEMOTHERAPY WITH PIRFENIDONE" FASEB JOURNAL (FEDERATION OF AMERICAN SOCIETIES FOR EXPERIMENTAL BIOLOGY), BETHESDA, US, vol. 41, no. 5, 1982, page 1550 XP008000826 ISSN: 0892-6638
- NICOD L P: "Pirfenidone in idiopathic pulmonary fibrosis" LANCET, XX, XX, vol. 354, no. 9175, 24 July 1999 (1999-07-24), pages 268-269, XP004266626 ISSN: 0140-6736
- KATSUMA SUSUMU ET AL: "Molecular monitoring of bleomycin-induced pulmonary fibrosis by cDNA microarray-based gene expression profiling" BIOCHEMICAL AND BIOPHYSICAL RESEARCH COMMUNICATIONS, ACADEMIC PRESS, SAN DIEGO, CA, US, vol. 288, no. 4, 9 November 2001 (2001-11-09), pages 747-751, XP002237871 ISSN: 0006-291X
- KAMINSKI N ET AL: "Global analysis of gene expression in pulmonary fibrosis reveals distinct programs regulating lung inflammation and fibrosis" PROCEEDINGS OF THE NATIONAL ACADEMY OF SCIENCES OF USA, NATIONAL ACADEMY OF SCIENCE. WASHINGTON, US, vol. 97, no. 4, 15 February 2000 (2000-02-15), pages 1778-1783, XP002237872 ISSN: 0027-8424

## Description

The present invention relates to a novel pharmaceutical kit and it's use for the improved treatment of idiopathic pulmonary fibrosis (IPF).

### BACKGROUND OF THE INVENTION

### Interstitial lung diseases

Reliable diagnosis of is of critical importance for disease management, research, epidemiology and development of specific therapies.

Interstitial lung diseases (ILD) are a heterogeneic group of chronic inflammatory reactions of the lung. Different forms of ILD are known which comprise, for example, of idiopathic pulmonary fibrosis (IPF), hypersensivity pneumonitis, scleroderma, Systemic Lupus Erythematosus, Rheumatoid Arthritis, Churg-Strauss syndrome, Wegener's granulomatosis, and Good-pasture Syndrome. This process is characterized by a combination of injury and exaggerated but futile attempts of tissue repair that transforms the regular maintenance of cellular growth into progressive development of scars. Characteristic features of this reaction are: repeated damage; intensified proteolytic activity and change in the composition of extracellular matrix (ECM) components. This combination leads to a shifted cellular immune response and a relentless induction of mesenchymal growth.

Cytokines, such as tumor necrosis factor-α, and mediators of growth, such as transforming growth factor β₁ (TGFβ₁), have long been implicated in this process. Of these mediators, TGFβ₁ has probably become the most important one, due to its strong activity to stimulate mesenchymal growth and its ability to modulate cellular immune functions. TGF-β₁ is known to cause severe pulmonary fibrosis when overexpressed in animal models *(*Sime PJ, Xing Z, Graham FL, Csaky KG, Gauldie J. Adenovector-mediated gene transfer of active transforming growth factor β1 induces prolonged severe fibrosis in rat lung. J Clin Invest 1997;100:768-76*.),* and a significant overexpression of this mediator is found in human pulmonary fibrosis *(*Broekelmann TJ, Limper AH, Colby TV, McDonald JA. Transforming growth factor beta 1 is present at sites of extracellular matrix gene expression in human pulmonary fibrosis. Proc Natl Acad Sci USA 1991;88:6692-6*.).* The immunomodulatory action of TGF-β₁ is well-known *(*Qin L, Ding Y, Bromberg JS. Gene transfer of transforming growth factor-beta 1 prolongs murine cardiac allograft survival by inhibiting cell-mediated immunity. Hum Gene Ther 1996; 7:1981-8*;* Gilbert KM, Thoman M, Bauche K, Pham T, Weigle WO. Transforming growth factor-beta 1 induces antigen-specific unresponsiveness in naive T cells. Immunol Invest 1997;26:459-72*;* Lawrence DA. Transforming growth factor-beta: a general review. Eur Cytokine Netw 1996;7:363-74*.)*. It includes the inhibition of interferon gamma release *(*Naganuma H, Sasaki A, Satoh E, Nagasaka M, Nakano S, Isoe S, et al. Transforming growth factor-beta inhibits interferon gamma secretion by lymphokine-activated killer cells stimulated with tumor cells. Neurol Med Chir Tokyo 1996; 36:789-95*.)*, the suppression of interferon gamma-dependent immune reactions *(*Lee YJ, Han Y, Lu HT, Nguyen V, Qin H, Howe PH, et al. TGF-beta suppresses interferon gamma induction of class II MHC gene expression by inhibiting class II transactivator messenger RNA expression. J Immunol 1997; 158:2065-75*.),* and the induction of immunosuppressive CD8+ lymphocytes *(*Gray JD, Hirokawa M, Horwitz DA, J Exp Med 180, 1937-1942, 1994*.).* Indeed, modulation of cellular immunity in patients with progressive fibrosis has been observed for years, even in forms, which clearly represent *different* mechanisms of initial damage. Recent investigations have demonstrated that progressive scarring in idiopathic pulmonary fibrosis is accompanied by a shift of the cytokine balance in T lymphocytes that favors the formation of the so-called T helper type 2 (Th2) reaction *(*Prior C, Haslam PL. In vivo levels and in vitro production of interferon gamma in fibrosing interstitial lung diseases. Clin Exp Immunol 1992;88:280-7*.;* Ziesche R, Kink E, Herold C, Podolsky A, Block LH. Therapy of chronic interstitial lung disease with a combination of Interferon gamma and low-dose prednisolone. Chest 1996; 110:25S.*)*. This reaction is characterized by an increase of 'Th2' cytokines, such as interleukin (ID) -4, IL-10 and IL-13, and a reduction or even a complete loss of interferon gamma, the main mediator of the T helper type 1 reaction *(*Majumdar S, Li D, Ansari T, et al. Tissue cytokine profiles of cryptogenic fibrosing alveolitis (CFA) and fibrosing alveolitis associated with systemic sclerosis (FASSc) are distinct: a quantitative in situ study of open lung biopsies. Eur Respir J 1999; 14: 251-7*.).* In bleomycin-induced lung fibrosis, transcription of the interferon gamma gene decreases from day 7 onwards and is no longer detectable on day 28. Reciprocally, transcription of IL-13, which also has fibrogenic activity, (Romagnani S. Th1/Th2 cells. Inflamm Bowel Dis. 1999; 5: 285-94*.*; Fallon PG, Richardson EJ, McKenzie GJ, McKenzie AN. Schistosome infection of transgenic mice defines distinct and contrasting pathogenic roles for IL-4 and IL-13: IL-13 is a profibrotic agent. J Immunol. 2000; 164 :2585-91*.),* begins to increase on day 7.

In contrast to the chronic inflammatory reaction in fibrosis, acute inflammation of the pulmonary interstitium as a result of infection with *M. pneumoniae* is characterized by a simultaneous transcription of both Th1 and Th2 cytokine genes, i.e. IL-12 and interferon gamma, and IL-4. In addition, this reaction includes an increased transcription of TGF-β1, probably as a sign of activated tissue repair.
Unfortunately, as a result of the usually late recognition of IPF, the early cellular events in this disease are virtually impossible to assess. However, analysis of mRNA accumulation for IL-4, interferon gamma, and TGF-β₁ in an individual with familial idiopathic pulmonary fibrosis, with symptoms of breathlessness on maximum exertion of less than a year, already demonstrates the shift of the immune balance and the intense activation of transcription of TGF-β₁. In the group of individuals with idiopathic pulmonary fibrosis, we found that the amount of mRNA accumulation of the TGF-β₁ gene was seven fold higher than that in normal lungs. In conclusion, our observations clearly demonstrate features of pathologically intensified repair mechanisms.
The intensity of repair is directly influenced by mechanisms of inflammation causing intensified turnover of both ECM and cellular components. As a result, chronic inflammation, usually induced by various infective agents, aggravates pathologic tissue repair.
Even if the causative agents were known, at present organ fibrosis cannot be sufficiently treated with any medication. Millions of people are dying from slow destruction of vital organ systems owing to pathological restructuring of functional organ tissue. This relentless process.is known as fibrosis or tissue remodeling that is primarily due to fibroproliferative mechanisms. The only remedy is organ transplantation, which is associated with numerous costly complications.
The fibroproliferative reaction concerns all organs of the body. In the gas-exchanging lung tissue it is known as lung fibrosis, in the bronchi it is known as bronchial asthma, in the liver as cirrhosis, in the kidney as glomerulosclerosis, in the general circulation as arteriosclerosis and coronary artery sclerosis, and in the pulmonary circulation as pulmonary hypertension.
These conditions are collectively known as fibroproliferative diseases. In fibrosis, healthy tissue is progressively replaced by components of the connective and supporting tissue of the human body. This process is based on the pathologically accelerated growth rate of tissue cells, which would normally accomplish regular wound healing. Thus, fibroproliferative diseases may be defined as uncontrollably accelerated wound healing. In fibrosis, the replacement of functional organ tissue continues until complete loss of organ function occurs. The currently available modes of diagnosis and treatment for all fibroproliferative diseases are inadequate.

### Interferon gamma (IFN-γ)

IFN-γ is a naturally glycoprotein cytokine, acting synergistically with other cytokines to exert its wide ranging effects. IFN-γ is a naturally occurring glycoprotein having a molecular weight of about 17.000 which can be commercially produced today also by recombinant techniques.
For improvement of the pharmacokinetic features, IFN-γ can be modified by pegylation to obtain PEG-IFN-γ. In addition to injection techniques, IFN-γ can be administered by inhalation to the lungs to diminish undesirable side effects. IFN-γ exhibits antiviral activity and together with other cytokines it plays an pivotal immunoregulatory role within the human immune system (an immunostimulating or an immunosuppressive effect, dependent on cell activation and location). Effects of IFN-γ include the induction of MHC class II antigens, macrophage activation, increased immunoglobulin production from B lymphocytes and enhanced NK cell activity.
The general antifibrotic properties of IFN-γ are well-documented *(*Lortat-Jacoh H, Kleinman HK, Grimaud JA. High-affinity binding of interferon-gamma to a basement membrane complex (matrigel) . J Clin Invest. 1991; 87: 878-83*. ;* Narayanan AS, Whithey J, Souza A, Raghu G. Effect of gamma-interferon on collagen synthesis by normal and fibrotic human lung fibroblasts. Chest. 1992 May;101(5):1326-31*;* Hjelmeland LM, Li JW, Toth CA, Landers MB 3d. Antifibrotic and uveitogenic properties of gamma interferon in the rabbit eye. Graefes Arch Clin Exp Ophthalmol. 1992;230(1):84-90*;* Hyde DM, Henderson TS, Giri SN, Tyler NK, Stovall MY. Effect of murine gamma interferon on the cellular responses to bleomycin in mice. Exp Lung Res. 1988;14 (5) :687-704*.).* It has already been demonstrated that IFN-γ causes a reduced expression of TGF-β₁ together with a reduction in the amount of fibrosis *(*Giri SN, Hyde DM, Marafino BJ Jr. Ameliorating effect of murine interferon gamma on bleomycin-induced lung collagen fibrosis in mice. Biochem Med Metab Biol. 1986 Oct; 36(2):194-7*.).* When using IFN-γ under clinical conditions, the transcription of TGF-β₁ and that of CTGF are significantly diminished after six months of therapy *(*Ulloa L, Doody J, Massague J. Inhibition of transforming growth factor-beta/SMAD signalling by the interferon-gamma/STAT pathway. Nature 1999; 397: 710-3*.) .* The simultaneous improvement of lung function in individuals suffering from IPF receiving IFN-γ provides additional support for the hypothesis that the mesenchymal activation in individuals with lung fibrosis depends, at least in part, on the continuous overexpression of TGF-β₁ and CTGF *(*Ziesche R, Hofbauer E, Wittmann K, Petkov V, Block LH. A preliminary study of long-term treatment with interferon gamma-1b and low-dose prednisolone in individuals with idiopathic pulmonary fibrosis. N Engl J Med. 1999; 341: 1264-9*;* EP0795332A2*.).* Moreover, these results suggest that an acquired deficiency of IFN-γ may be one reason for the exaggerated wound healing process characteristic of progressive organ fibrosis.
IFN-γ was shown to be the first clinically applicable anti-fibrotic drug. As any endogenous substance, IFN-γ has pleiotropic effects dependent on the existing conditions within the body. In case of an exaggerated, yet non- or low-inflammatory wound healing, such as in IPF, the drug exerts powerful highly beneficial anti-fibrotic properties. However, in the presence of accompanying infections, such as chronic bacterial, viral or fungal infections, the drug will add to the inflammatory process and even reinforce, by intensifying tissue repair, the fibrotic process.

Thus, for a safe and effective use of the drug, it is necessary to molecularly measure the state of tissue response prior to application of the drug. The absence of the mRNA transcription of the gene for IFN-γ in the presence of intensified transcription of factors of wound healing may serve as the first vague discriminator between fibrosis as a result of deregulated wound healing itself, versus fibrosis as a result of intensified inflammation. The first attempts to analyze gene expression patterns in pulmonary fibrosis in animal models demonstrate the power of this tool to discriminate between the fibrotic process itself and accompanying mechanisms *(*Kaminski et al., Proc Natl Acad Sci U S A 2000 Feb 15;97(4):1778-83 Global analysis of gene expression in pulmonary fibrosis reveals distinct programs regulating lung inflammation and fibrosis*;* Katsuma et al., Biochem Biophys Res Commun 2001 Nov 9;288(9):797-51 Molecular monitoring of bleomycin-induced pulmonary fibrosis by cDNA microarray-based gene expression profiling*).* A medication which combines the molecular diagnosis (transcription analysis in diseased ILD patients compared to control populations - healthy individuals or patients suffering from different lung diseases - with a distinct quantitative, specific expression difference to those) with proven effectiveness of a antifibrotic drug like IFN-γ or pirfenidone under clinical conditions provides the essential step for the first bio-medical based treatment of these multifunctional diseases.

To sum up, the real effectiveness of IFN-γ therapy mainly depends on four conditions:
(a) the proof of IFN-γ deficiency in pulmonary tissue;
(b) the control of infections or other inflammatory conditions, either pre-existing or acquired during therapy;
(c) the gene expression profiling of the patients suspected to have an interstitial lung disease;
(d) the duration of disease-related symptoms, reflecting the extent of completed scarring.

Clinical experience suggests that repeated phases of increased inflammation due to a large variety of infectious agents are the most common reason for a failure of IFN-γ in the treatment of ILD. Thus, clinical and molecular control of inflammation and infections, especially in the peripheral bronchi is crucial for a success of an anti-fibrotic treatment with IFN-γ.

Thus it is goal of the work of the present invention to improve the treatment of IPF using IFN-γ in pharmaceutical composition with a diagnostic array of candidate polynucleotides.

### Disease-related gene expression profiles

With the completion of the Human Genome Project sequencing, biomedical research will be revolutionised by the ability to carry out investigations on a genome wide scale:
Arch Ophthalmol 2001 Nov;119(11):1629-34 Microarray analysis of gene expression in human donor corneas**.**
Jun AS et al. Ann Surg 2001 Dec;239(6):769-78; discussion 778-9 Identification of disease-specific genes in chronic pancreatitis using DNA array technology.
Friess H et al. DNA Cell Biol 2001 Nov;20(11):683-95 A gene expression profile of Alzheimer's disease**.**
Loring JF et al.Pharmacogenomics J 2001;1(4):272-87 Molecular classification of psoriasis disease-associated genes through pharmacogenomic expression profiling.
   Oestreicher JL et al.

Gene expression technology is gaining increasingly widespread use as a means to determine the expression of potentially all human genes at the level of messenger RNA. Gene specific sequences (oligo-, polynucleotides or cDNAs) are immobilised on arrays, hybridised with complex probes represented by a mixture of cDNAs of respective samples from human biopsies and other human materials (reversely transcribed from messenger RNA), and labelled with different dyes. Hybridised slides are analysed with sensitive scanning methodologies. Genechips containing gene sequences deliver fast and excellent overview of the expression pattern of the biological samples. Thus, the genome-wide gene expression analysis provides new insights into causes of disease, and elucidates the as yet unknown biological gene expression variations between seemingly similar diseases in defined detection ranges, leading to a new classification system valuable in accurate diagnosis.

Important applications include:
(a) development of a more global understanding of the qualitative and quantitative gene expression profiles that contribute to disease processes;
(b) discovery of new diagnostic and prognostic indicators and biomarkers of therapeutic response;
(c) identification and validation of new molecular targets for drug development;
(d) provision of an improved understanding of the molecular mode of action during lead identification and optimisation;
(e) designing more specific and efficient treatment strategies;
(f) prediction of potential side-effects during development and toxicology studies;
(g) confirmation of molecular modes of action during hypothesis-testing clinical trials;
(h) identification of genes involved in conferring drug sensitivity and resistance;
(i) prediction of patients most likely to benefit from the drug and use in general pharmacogenomic studies.

As a result of further technological improvements, array-based genechips will become essential part for clinical interventions in identifying and quantifiying individual gene-expression patterns for purposes of patient-oriented therapy, and in establishing a method for predicting efficacy of drugs for individual patients, given the functional complexity of most diseases, especially those involving fibroproliferative processes. Considering the fact that inflammatory diseases reflect qualitative and quantitative changes in the activity of certain genes and/or gene clusters, the molecular assessment will allow for a higher specificity and efficacy of medical treatments and will be considered integral part of the therapeutic composition.
To achieve this goal, new molecular markers and markers of progression are needed for improved staging and for better assessment of diagnosis and treatment of complex diseases. Gene expression profiling techniques in combination with therapeutics offer the opportunity to discover such patho-physiologically relevant markers of disease.

In the context of this disclosure, a number of terms shall be utilized.
The term "polynucleotide" refers to a polymer of RNA or DNA that is single-stranded, optionally containing synthetic, non-natural or altered nucleotide bases. A polynucleotide in the form of a polymer of DNA may be comprised of one or more segments of cDNA, genomic DNA or synthetic DNA.
The term "subsequence" refers to a sequence of nucleic acids that comprises a part of a longer sequence of nucleic acids. The term "immobilized on a support" means bound directly or indirectly thereto including attachment by covalent binding, hydrogen bonding, ionic interaction, hydrophobic interaction or otherwise.

Polynucleotide arrays allow to qualitatively and quantitatively study mRNA expression levels of selected candidate genes in human materials.

Polynucleotide or DNA arrays consist of large numbers of DNA molecules spotted in a systematic order on a solid support or substrate such as a nylon membrane, glass slide, glass beads or a silicon chip. Depending on the size of each DNA spot on the array, DNA arrays can be categorized as microarrays (each DNA spot has a diameter less than 250 microns) and macroarrays (spot diameter is grater than 300 microns). When the solid substrate used is small in size, arrays are also referred to as DNA chips. Depending on the spotting technique used, the number of spots on a glass microarray can range from hundreds to tens of thousands.

DNA microarrays serve a variety of purposes, including gene expression profiling, de novo gene sequencing, gene mutation analysis, gene mapping and genotyping. cDNA microarrays are printed with distinct cDNA clones isolated from cDNA libraries. Therefore, each spot represents an expressed gene, since it is derived from a distinct mRNA.
Typically, a method of monitoring gene expression involves providing
(1) a pool of sample polynucleotides comprising RNA transcripts of target genes or nucleic acids derived from the RNA transcripts;
(2) hybridizing the sample polynucleotides or nucleic acids derived from the RNA transcripts to an array of probes (for example, polynucleotides obtained from a polynucleotide library including control probes, and
(3) detecting the hybridized double-stranded polynucleotides/nucleic acids. The label used to mark polynucleotide samples is selected from the group consisting of radioactive, colorimetric, enzymatic, molecular amplification, bioluminescent or fluorescent label.

The present disclosure also to any polynucleotide library as previously described wherein said polynucleotides are immobilized on a solid support in order to form a polynucleotide array.
Preferably the support is selected from the group consisting of a nylon membrane, glass slide, glass beads, or a silicon chip.
The present disclosure relates to a polynucleotide library useful in the molecular characterization of Idiopathic Pulmonary Fibrosis (IPF), the library including a pool of polynucleotide sequences or subsequences thereof wherein the sequences or subsequences are either underexpressed or overexpressed in IPF diseased cells. Preferably, a set of sequences is selected from oligo- or polynucleotide probes having a sequence defined by, or correlated to, or derived from the group of genes consisting of candidate genes indicated in the following list, representing increased gene expression levels of IPF cells versus cells from patients with a different lung disorder:
NM_007337.1
mucin 4, tracheobronchial, AJ242547.1, (SEQ. ID NO:6)
mucin 5, subtype B, tracheobronchial, AI697108, (SEQ ID NO:12)
myosin regulatory light chain 2, smooth muscle isoform, J02854.1, (SEQ ID NO:19)
insulin-like growth factor binding protein 2 (36kD), BC004312.1, (SEQ ID NO:23)
cadherin 1, type 1, E-cadherin (epithelial), L08599.1, (SEQ ID NO:29)
connective tissue growth factor, M92934.1, (SEQ ID NO:50)
keratin 15, BC002641.1, (SEQ ID NO:71)
cytokeratin 17, Z19574, (SEQ ID NO:118)
matrix metalloproteinase 7 (matrilysin, uterine), BC003635.1, (SEQ ID NO:82)
cytokeratin 4: X07695.1, (SEQ ID NO:116)
insulin-like growth factor 1 receptor: NM_000875.2, (SEQ ID NO:148)
keratin 5 (epidermolysis bullosa simplex, Dowling-MearaKobnerWeber-Cockayne types): M21389.1, (SEQ ID NO:149),
transforming growth factor β₁: NM_000660, (SEQ ID NO:151)
wherein each oligo- or polynucleotide probe is obtainable on the basis of structural information mediated by the sequence data provided by the respective accession number set out for each candidate gene.

Yet another set of sequences is selected from oligo- or polynucleotide probes having a sequence defined by, or correlated to, or derived from the group of genes consisting of candidate genes indicated in the following list, representing decreased gene expression levels of IPF cells versus cells from patients with different lung disorder:
surfactant, pulmonary-associated protein C, BC005913.1, (SEQ ID NO:156)
interferon, gamma-inducible protein 30, AF097362.1, (SEQ ID NO:181)
interleukin 7 receptor, M29696.1, (SEQ ID NO:214)
pulmonary surfactant protein (SP5), J03553, (SEQ ID NO:222)
surfactant, pulmonary-associated protein A2, NM_006926.1, (SEQ ID NO:227),
interleukin 13 receptor, alpha 2: U70981.1, (SEQ ID NO:257)
interferon-gamma-inducible indoleamine 2,3-dioxygenase (IDO): M4455.1, (SEQ ID NO:276)
relaxin 1 (H1) : BC005956.1, (SEQ ID NO:313)
   wherein each oligo- or polynucleotide probe is obtainable on the basis of structural information mediated by the sequence data provided by the respective accession number set out for each candidate gene.

For example, suitable sequences which may be used for oligonucleotide design may be downloaded from the NCBI GenBank (http://www.ncbi.nlm.gov/GenBank/index.html) using the Accession number listed behind the gene name.

All accession numbers give access to mRNA sequences; since the cDNA of the patients is reverse transcribed, the oligonucleotide have to sense strand and are therefore identical with the mRNA sequence. Preferably the pool of polynucleotide sequences or subsequences correspond substantially to the polynucleotide sequences useful in qualitative and quantitative differentiating a normal cell, or a cell from a patient suffering from non-fibrosing lung disease, from a cell of an patient with Idiopathic Pulmonary Fibrosis (IPF).

The invention concerns the part of pharmaceutical composition for detecting differentially expressed polynucleotide sequences which are correlated with IPF, said part comprising:
a) obtaining a polynucleotide sample from a patient; and b) reacting the sample polynucleotide obtained in step (a) with a probe immobilized on a solid support wherein said probe comprises any of the polynucleotide sequences of the libraries previously described or an expression product encoded by any of the polynucleotide sequences of said libraries and c) detecting the reaction product of step (b) as a prerequisite for a subsequent administration of suitable drug.

In one instance of the present disclosure, the detection of differentially expressed polynucleotide sequences is used for detecting, diagnosing, staging, monitoring, prognosticating, preventing or treating conditions associated with ILD, and namelly idiopathic pulmonary fibrosis IPF.

The detection of differentially expressed polynucleotide sequences is particular useful wherein the product encoded by any of the polynucleotide sequences or subsequences is involved in a receptor-ligand reaction on which detection is based.

Differences in gene expression are accepted as different when the signals from patient material are at least two fold lower or higher than those from comparative material, be it from healthy volunteers material or from other diseased material. This threshold is commonly accepted for the evaluation of expression arrays.

### SUMMARY OF THE INVENTION

It was surprisingly found that a pharmaceutical composition of interferon gamma together with a gene expression analysis of the patients with lung diseases results in a faster and more successful treatment of idiophathic pulmonary fibrosis (IPF). Thus, the invention relates to

The present invention is defined by the claims.

A pharmaceutical kit comprising:
(i) a package comprising interferon gamma, optionally together with a pharmaceutically acceptable carrier, diluent or excipient for use the treatment of idiopathic pulmonary fibrosis, the successful pre-treatment of underlying infections, wherein the patien treated has a pool of polynucleotide sequences or subsequences which are either underexpressed or overexpressed in idiopathic pulmonary fibrosis cells compared to other lung disorders,
   wherein the overexpressed sequences or subsequences are mucin 4, tracheobronchial, AJ242547.1 (SEQ ID NO:6);
   mucin 5, subtype B, tracheobronchial, AI697108 (SEQ ID NO:12); myosin regulatory light chain 2, smooth muscle isoform, J02854.1 (SEQ ID NO:19);
   insulin-like growth factor binding protein 2 (36kD), BC004312.1 (SEQ ID NO:23);
   cadherin 1, type 1, E-cadherin (epithelial), L08599.1 (SEQ ID NO:29); connective tissue growth factor, M92934.1 (SEQ ID NO:50);
   keratin 15, BC002641.1 (SEQ ID NO:71);
   cytokeratin 17,Z19574 (SEQ ID NO:118);
   matrix metalloproteinase 7 (matrilysin, uterine), BC003635.1 (SEQ ID NO:82);
   cytokeratin 4: X07695.1 (SEQ ID NO:116);
   insulin-like growth factor 1 receptor: NM_000875.2 (SEQ ID NO:148); keratin 5 (epidermolysis bullosa simplex, Dowling-MearaKobnerWeber-Cockayne types): M21389.1 (SEQ ID NO:149);
   transforming growth factor β₁, NM_000660 (SEQ ID NO:151);
   and the underexpressed sequences or subsequences are surfactant, pulmonary-associated protein C, BC005913.1 (SEQ ID NO:156);
   interferon, gamma-inducible protein 30, AF097362.1 (SEQ ID NO:181); interleukin 7 receptor, M29696.1 (SEQ ID NO:214);
   pulmonary surfactant protein (SP5), J03553 (SEQ ID NO:222); surfactant, pulmonary-associated protein A2, NM_006926.1 (SEQ ID NO:227);
   interleukin 13 receptor, alpha 2: U70981.1 (SEQ ID NO:257); interferon-gamma-inducible indoleamine 2,3-dioxygenase (IDO):
   M34455.1 (SEQ ID NO:276);
   relaxin 1 (H1): BC005956.1 (SEQ ID NO:313), characterised in that the treatment comprises the diagnostic step of determining whether or not the patient has the above stated pool of polynucleotide sequences or subsequences which are either under- or overexpressed in IPF.

Furthermore, the use of the pharmaceutical kit for the manufacture of a medicament for the treatment of idiopathic pulmonary fibrosis comprising
(i) a package comprising interferon gamma, optionally together with a pharmaceutically acceptable carrier, diluent or excipient as claimed in claim 1.

To sum up and in more detail, the present disclosure comprising the invention relates to the following topic: use of
A pharmaceutical kit comprising
   (i) a package comprising IFN-γ, or PEG-IFN-γ, as defined in claim 1 (ii) for the manufacture of a medicament for the treatment of idiopathic pulmonary fibrosis (IPF) art the for use or treating IPF. Further disclosed is:
      - a corresponding use, wherein IFN-γ is administered to the individual in a single dose amount varying from 1.0 to 5.0 µg / kg body weight; and
      - a corresponding use, wherein the glucocorticoid compound is administered to the individual in a single dose amount varying from 100 to 350 µg / kg body weight; and
      - a corresponding use, wherein IFN-γ is administered to the individual by inhalation after a gene expression analysis.

### DETAILED DESCRIPTION

Suitable compounds which have the therapeutic effect within the combination according to the invention, are, interferon gamma or pegylated interferon gamma in combination with the gene expression analysis of diseased patients.

The term **"stabilized form"** means a derivative or analogue wherein the parent drug was altered in order get more stability and increased half-life in blood and serum. Polypeptides and proteins may be protected against proteolysis by the attachment of chemical moieties. Such attachment may effectively block the proteolytic enzyme from physical contact with the protein backbone itself, and thus prevent degradation. Polyethylene glycol is one such chemical moiety which has been shown to protect against proteolysis (Sada, et al., J. Fermentation Bioengineering 71: 137-139, 1991). In addition to protection against proteolytic cleavage, chemical modification of biologically active proteins has been found to provide additional advantages under certain circumstances, such as increasing the stability and circulation time of the therapeutic protein and decreasing immunogenicity. (US. 4,179,337; Abuchowski et al., Enzymes as Drugs.; J.S. Holcerberg and J. Roberts, eds. pp. 367-383, 1981; Francis, Focus on Growth Factors 3: 4-10; EP 0 401 384). The addition of polyethylene glycol increases stability of the peptides and polypeptides of this invention at physiological pH as compared to non-pegylated compounds. The pegylated polypeptide /protein is also stabilized with regard to salts.

The term **"fusion protein"** means a compound, especially a stabilized form, consisting of a polypeptide according to the invention, preferably interferon gamma, which is fused to another peptide or protein.

The term **"pharmaceutical kit"** means a package comprising two or more packages or containers containing one or more, preferably one pharmaceutically active compound or agent and a gene expression analysis device, wherein the agent or compound in said packages or containers are administered to an individual after gene expression analysis is performed.

The pharmaceutical compositions according to the invention comprising interferon gamma and a gene expression analysis as defined above and below can be used as medicament for the treatment of an individual.

The term "**individual**" preferably refers to mammals, especially humans.

The compound according to this invention, IFN-γ is used in a pharmaceutical formulations, comprising, as a rule, a pharmaceutically acceptable carrier, excipient or diluents. Techniques for the formulation and administration of the compounds of the present invention may be found in "Remington's Pharmaceutical Sciences" Mack Publishing Co., Easton PA.

As used herein, the term **"pharmaceutically acceptable carrier"** means an inert, non toxic solid or liquid filler, diluent or encapsulating material, not reacting adversely with the active compound or with the individual, or any other formulation such as tablets, pills, dragees, capsules, gels, syrups, slurries, suspensions and the like. Suitable, preferably liquid carriers are well known in the art such as sterile water, saline, aqueous dextrose, sugar solutions, ethanol, glycols and oils, including those of petroleum, animal, vegetable, or synthetic origin, for example, peanut oil, soybean oil and mineral oil. Tablets and capsules for oral administration contain conventional excipients such as binding agents, fillers, diluents, tableting agents, lubricants, disintegrants, and wetting agents. The tablets may be coated according to methods well known in the art.

The formulations may be administered as unit doses containing conventional non-toxic pharmaceutically acceptable carriers, diluents, adjuvants and vehicles which are typical for parenteral administration.

The term **"parenteral"** includes herein subcutaneous, intravenous, intra-articular and intratracheal injection and infusion techniques. Parenteral compositions and combinations are most preferably administered intravenously either in a bolus form or as a constant fusion according to known procedures.

Also other administrations such as oral administration or administration by inhalation or nasal spray are also object of the invention. Inhalation of vapors containing interferon gamma as specified is also a preferred way of administration. For inhalations the compound is preferably brought in an aerosol form. Aerosols and techniques to make them are well known in the art. Aerosols applicable by inhalers containing interferon gamma are preferred if direct pulmonary symptoms have to be treated.

Unit doses according to the invention may contain daily required amounts of the compound according to the invention, or sub-multiples thereof to make up the desired dose. The optimum therapeutically acceptable dosage and dose rate for a given individual (mammals, including humans) depends on a variety of factors, such as the activity of the specific active material employed, the age, body weight, general health, sex, diet, time and route of administration, rate of clearance, enzyme activity, the object of the treatment, i. e., therapy or prophylaxis and the nature of the disease to be treated. Therefore, in the pharmaceutical compositions according to the invention for the therapy of an individual, a pharmaceutical effective daily dose of the respective compound in said composition is:
Interferon gamma (IFN-γ): It could be shown that interferon-γ is effective in the combination therapy according to the invention in a dose of 1.0 - 5.0 µg / kg body weight, preferably 2.0 - 3.0 µg / kg body weight, 1- 5 times per week. The above-indicated single dosages of interferon-γ are administered parenteral, preferably subcutaneously to the patient.
The doses of glucocorticoids which can be administered optionally together with IFN-γ to an individual vary according to the invention from 10 - 100 mg / single dose and more preferably from 15 - 80 mg, which corresponds to approximately 100 - 350 µg /kg body weight, preferably 100-150 µg /kg body weight.

### SHORT DESCRIPTION OF THE FIGURES

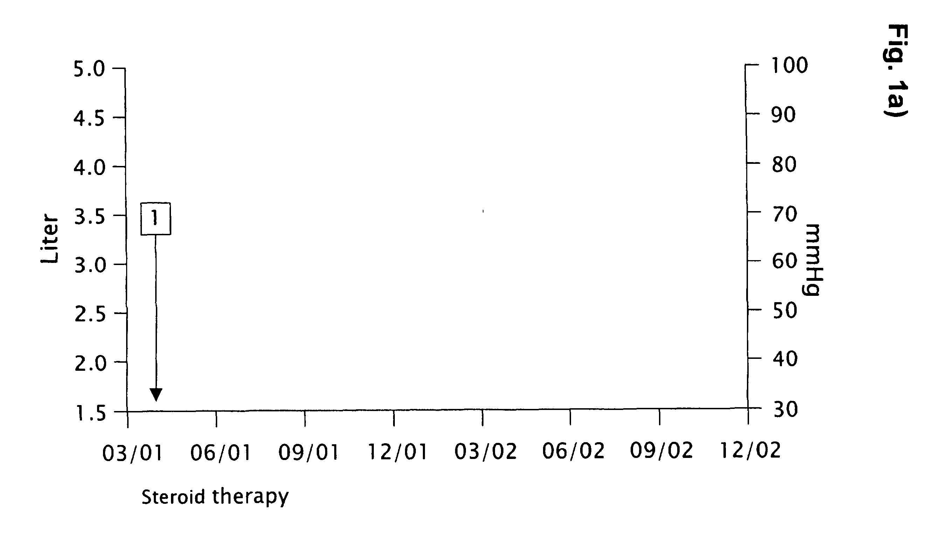

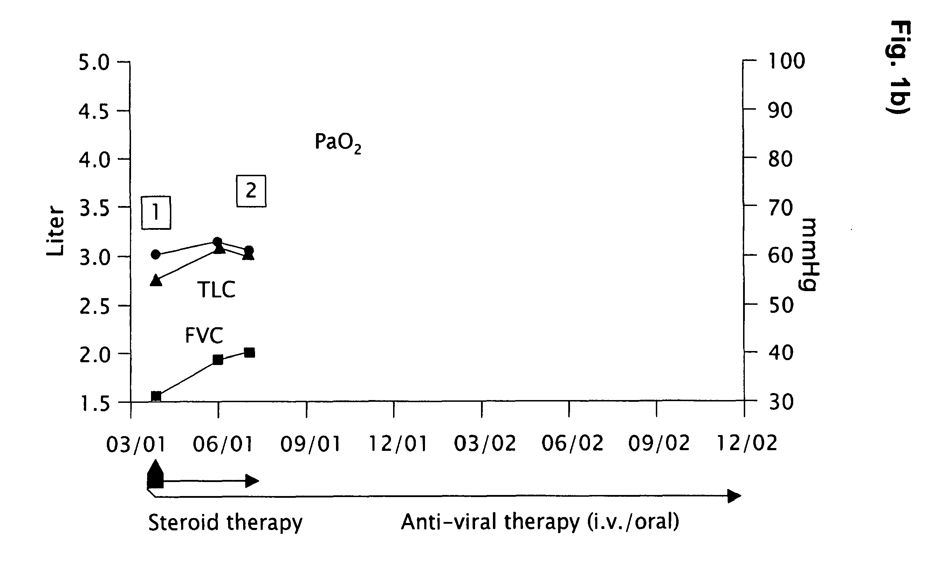

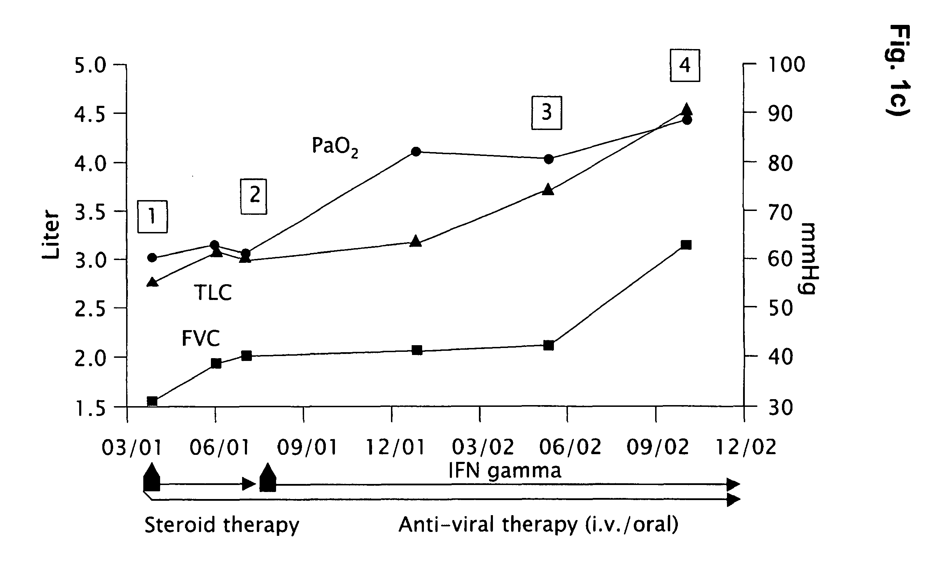

Fig. 1: Figure 1 depicts the total lung capacity (TLC), the partial pressure of arterial oxygen (PaO2), and the forced vital capacity (FVC) of a patient with histologically proven UIP (open lung biopsy) and failure of immunosuppressive treatment (f, 59 yr) treated with interferon gamma.

### EXAMPLES

Biopsies were taken from patients suffering from severe interstitial lung disease (ILD), after informal consent was given, using the surgical method of bronchoscopy. Obtained tissue probes were stored and processed for isolation of total RNA in RNA*later*™ (patent pending), an aqueous, non-toxic tissue storage reagent that stabilizes and protects cellular RNA in intact, unfrozen tissue samples.
The dissected tissue (less than 0.5 cm in any one dimension) is submerged in approximately 5 volumes of RNAlater (e.g., a 0.5 g sample requires about 2.5 ml of RNAlater) at room temperature. The solution permeates the cells, stabilizing the RNA. Then, RNA is isolated using the one-step RNA isolation methods, such as TRIzol® Reagent (Life Technologies), following the instructions of the supplier and finally eluted in H₂O.

### Preparation of Labeled cRNA and Hybridization to Microarrays.

Double-stranded cDNA was synthesized out of the isolated patients RNA samples using a cDNA synthesis kit (Superscript; Life Technologies) employing oligo(dT) priming. The resulting cDNA was used for *in vitro* transcription (Ambion T7 Megascript system) in the presence of biotin-11-CTP and biotin-16-UTP (Enzo Diagnostics). A total of 25-50 µg of the cRNA product in buffer [40 mM Tris·acetate (pH 8.1)/100 mM potassium acetate/30 mM magnesium acetate] was fragmented at 94°C for 35 min. It was then used as a hybridization probe from each patient for hybridization as recommended (Affymetrix, Santa Clara, CA).
Aliquots of the hybridization patients cRNA mixtures (10 µg cRNA in 200 µl hybridization mix) were hybridized to a Human Genome U133A Array. Each array was washed and scanned (Hewlett Packard, GeneArray scanner G2500A) according to procedures developed by manufacturer (Affymetrix).

**Analysis of GENECHIP Data.** Scanned output files were visually inspected for hybridization artifacts and then analyzed with GENECHIP 3.1 software (Affymetrix).
The expression analysis files created by GENECHIP 3.1 software were transferred to a database (Microsoft Access) and linked to Internet genome databases (e.g., NHLBI, or Swiss Prot).

**Analysis of Infectious agents in biopsies.** As patients with ILD repeatedly suffer from inflammations, biopsied material was tested for the presence of infectious agents. Most dominant was the observation of underlying Herpes virus infections (treated with Ganciclovir) and various bacterial infections (treated with various antibiotics), or few fungal infections.

**Therapy.** Therapy with interferon gamma was only started after successful pretreatment of the underlying infections and the assessment of the gene expression of the endogenous disease signiture genes. Application of molecular gene expression analysis followed by interferon gamma, led to a surprising, statistically highly significant increase of total lung capacity that was accompanied by a substantial increase of physical exertion capacity, statistically significant increased partial pressure of arterial oxygen at rest and a strong decrease of disease symptoms. This indicates that the pharmaceutical composition of gene expression analysis followed by interferon gamma is able to counterbalance fibrosing reactions in interstitial lung diseases and provides significant survival benefit to the patients with IPF.
Fig. 1a) depicts start data #1 of the treatment. 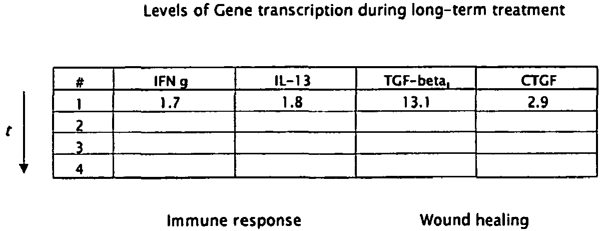
Fig. 1b) depicts the total lung capacity (TLC), the partial pressure of arterial oxygen (PaO2), and the forced vital capacity (FVC) of the same patient with histologically proven UIP (open lung biopsy) and failure of immunosuppressive treatment (f, 59 yr) between time #1 and time #2. 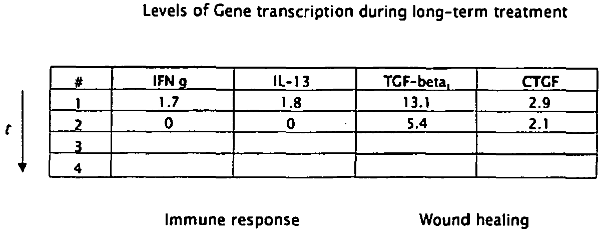
Fig. 1c) depicts the same data for the same patient between time #1 and time #4.

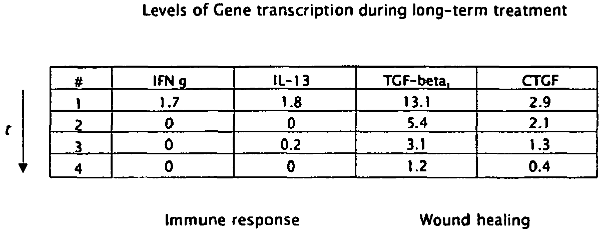

Pretreatment of CMV followed by selected gene transcription analysis and subsequent interferon gamma treatment lead to stabilization of the patient with a deadly disease.

## Claims

1. Use of a pharmaceutical kit for the manufacture of a medicament for the treatment of idiopathic pulmonary fibrosis after the successfull pre-treatment of underlying infections, comprising
(i) a package comprising interferon gamma, optionally together with a pharmaceutically acceptable carrier, diluent or excipient, wherein the patient has a pool of polynucleotide sequences or subsequences which are either underexpressed or overexpressed in idiopathic pulmonary fibrosis cells compared to other lung disorders,
wherein the overexpressed sequences or subsequences are mucin 4, tracheobronchial (SEQ ID NO:6);
mucin 5, subtype B, tracheobronchial (SEQ ID NO:12);
myosin regulatory light chain 2, smooth muscle isoform, (SEQ ID NO:19);
insulin-like growth factor binding protein 2 (36kD), (SEQ ID NO:23); cadherin 1, type 1, E-cadherin (epithelial) (SEQ ID NO:29); connective tissue growth factor (SEQ ID NO:50);
keratin 15 (SEQ ID NO:71);
cytokeratin 17 (SEQ ID NO:118);
matrix metalloproteinase 7 (matrilysin, uterine) (SEQ ID NO:82); cytokeratin 4 (SEQ ID NO:116);
insulin-like growth factor 1 receptor (SEQ ID NO:148);
keratin 5 (epidermolysis bullosa simplex, Dowling-MearaKobnerWeber-Cockayne types): (SEQ ID NO:149); transforming growth factor β₁ (SEQ ID NO:151);
and the underexpressed sequences or subsequences are surfactant, pulmonary-associated protein C (SEQ ID NO:156); interferon, gamma-inducible protein 30 (SEQ ID NO:181); interleukin 7 receptor (SEQ ID NO:214);
pulmonary surfactant protein (SP5) (SEQ ID NO:222);
surfactant, pulmonary-associated protein A2 (SEQ ID NO:227); interleukin 13 receptor, alpha 2 (SEQ ID NO:257); interferon-gamma-inducible indoleamine 2,3-dioxygenase (IDO) (SEQ ID NO:276);
relaxin 1 (H1) (SEQ ID NO:313), **characterised in that** the treatment comprises the diagnostic step of determining whether or not the patent has the above states pool of under- or overexpressed polynucleotide sequences or subsequences in IPF.

2. Use of interferon gamma according to claim 1, together with a glucocorticoid compound.

3. Use according to claim 1, wherein the interferon gamma is pegylated interferon gamma.

4. Use of pegylated interferon gamma according to claim 3, together with a glucocorticoid compound.

5. Use of pharmaceutical kit according to any of the preceding claims, wherein IFN-γ is administered to the individual in a single dose amount varying from 1.0 to 5.0 µg / kg body weight.

6. Use of pharmaceutical kit according to claim 2, wherein the glucocorticoid compound is administered to the individual in single dose amount *varying from* 100 to 350 µg / kg body weight.

7. Use of pharmaceutical kit according to claim 1, wherein interferon gamma is administered to the individual by inhalation.

8. A pharmaceutical kit comprising:
(i) a package comprising interferon gamma, optionally together with a pharmaceutically acceptable carrier, diluent or excipient for use in the treatment of idiopathic pulmonary fibrosis, after the successful pre-treatment of underlying infections wherein the patient has a pool of pool of polynucleotide sequences or subsequences which are either underexpressed or overexpressed in idiopathic pulmonary fibrosis cells compared to other lung disorders,
*wherein the overexpressed* sequences or subsequences are mucin 4, tracheobronchial (SEQ ID NO:6);
mucin 5, subtype B, tracheobronchial (SEQ ID NO:12);
myosin regulatory light chain 2, smooth muscle isoform (SEQ ID NO:19);
insulin-like growth factor binding protein 2 (36kD) (SEQ ID NO:23); cadherin 1, type 1, E-cadherin (epithelial) (SEQ ID NO:29); connective tissue growth factor (SEQ ID NO:50);
keratin 15 (SEQ ID NO:71);
cytokeratin 17 (SEQ ID NO:118);
matrix metalloproteinase 7 (matrilysin, uterine) (SEQ ID NO:82); cytokeratin 4 (SEQ ID NO:116);
insulin-like growth factor 1 receptor (SEQ ID NO:148);
keratin 5 (epidermolysis bullosa simplex, Dowling-MearaKobnerWeber-Cockayne types) (SEQ ID NO:149); transforming growth factor β₁ (SEQ ID NO:151);
and the underexpressed sequences or subsequences are *surfactant,* pulmonary-associated protein C (SEQ ID NO:156); interferon, gamma-inducible protein 30 (SEQ ID NO:181); interleukin 7 receptor (SEQ ID NO:214);
pulmonary surfactant protein (SP5) (SEQ ID NO:222);
surfactant, pulmonary-associated protein A2 (SEQ ID NO:227); interleukin 13 receptor, alpha 2 (SEQ ID NO:257); interferon-gamma-inducible indoleamine 2,3-dioxygenase (IDO) (SEQ ID NO:276);
relaxin 1 (H1) (SEQ ID NO:313), **characterised in that** the treatment comprises the diagnostic step of determining whether or not the patient has the above stated pool of under- or overexpressed polynucleotide sequences or subsequences in IPF.

9. The kit for use according to claim 8, also comprising a glucocorticoid compound.

10. The kit for use according to claim 8, wherein the interferon gamma is pegylated interferon gamma.

11. The kit for use according to claim 10, also comprising a glucocorticoid compound.

12. The kit for use according to claim 8, wherein IFNgamma is provided in single dose amount varying from 1.0 to 5.0 µg / kg body weight.

13. The kit for use according to claim 9, wherein the glucocorticoid compound is provided in single dose amounts varying from 100 to 350 µg / kg body weight.

14. The kit for use according to claim 8, wherein interferon gamma is suitable for administration by inhalation.

## Patentansprüche

1. Verwendung eines pharmazeutischen Kits zur Herstellung eines Medikaments zur Behandlung von idiopathischer pulmonarer Fibrose, umfassend
(i) interferon-gamma, gegebenenfalls zusammen mit einem pharmazeutisch annehmbaren Träger, Verdünnungsmittel oder Hilfsstoff
(ii) ein Paket umfassend eine Polynukleotid-Bibliothek für die molekulare Charakterisierung der idiopathischen pulmonaren Fibrose, wobei die Bibliothek einen Pool von Polynucleotid-Sequenzen oder Subsequenzen umfasst, worin genannte Sequenzen oder Subsequenzen entweder unterexprimiert oder überexprimiert sind in idiopathisch pulmonaren Fibrosezellen verglichen mit anderen Lungenerkrankungen, wobei die überexprimierten Sequenzen oder Subsequenzen mucin 4, tracheobronchial, AJ242547.1; mucin 5, subtype B, tracheobronchial, A1697108; myosin regulatory light chain 2, smooth muscle isoform, J02854.1; insulin-like growth factor binding protein 2 (36kD), BC004312.1; cadherin 1, type 1, E-cadherin (epithelial), L08599.1; connective tissue growth factor, M92934.1; keratin 15, BC002641.1; cytokeratin 17, Z19574; matrix metalloproteinase 7 (matrilysin, uterine), BC003635.1; cytokeratin 4: X07695.1; insulin-like growth factor 1 receptor: NM_000875.2; keratin 5 (epidermolysis bullosa simplex, Dowling-MearaKobnerWeber-Cockayne types): M21389.1; transforming growth factor b₁, NM_000660 sind, und die unterexprimierten Sequenzen oder Subsequenzen surfactant, pulmonary-associated protein C, BC005913.1; interferon, gamma-inducible protein 30, AF097362.1; interleukin 7 receptor, M29696.1; pulmonary surfactant protein (SP5), J03553; surfactant, pulmonary-associated protein A2, NM_006926.1; interleukin 13 receptor, alpha 2: U70981.1; interferon-gamma-inducible indoleamine 2,3-dioxygenase (IDO): M34455.1; relaxin 1 (H1): BC005956.1. sind,
wobei das interferon gamma nach molekularer Charakterisierung der idiopathischen pulmonaren Fibrose, sowie nach erfolgreicher Vorbehandlung der zugrunde liegenden Infektionen angewendet wird.

2. Verwendung von interferon gamma gemäss Anspruch 1, umfassend eine Glucocorticoid-Verbindung.

3. Verwendung gemäss Anspruch 1, wobei das interferon gamma pegyliertes interferon gamma ist.

4. Verwendung von pegyliertem interferon gamma gemäss Anspruch 3, umfassend eine Glucocorticoid-Verbindung.

5. Verwendung des pharmazeutischen Kits gemäss einem der vorhergehenden Ansprüche, wobei das interferon gamma dem Einzelnen in einzelner variablen Dosis von 1,0 bis 5,0 microg / kg Körpergewicht verabreicht wird.

6. Verwendung des pharmazeutischen Kits gemäss Anspruch 2, wobei die Glucocorticoid-Verbindung dem Einzelnen in einzelner variablen Dosis von 100 bis 350 microg / kg Körpergewicht verabreicht wird.

7. Verwendung des pharmazeutischen Kits gemäss Anspruch 1, wobei das interferon gamma dem Einzelnen durch Inhalierung zu verabreichen ist.

8. Pharmazeutische Zusammensetzung, umfassend
(i) ein Paket umfassend interferon-gamma, gegebenenfalls zusammen mit einem pharmazeutisch annehmbaren Träger, Verdünnungsmittel oder Hilfsstoff
(ii) ein Paket umfassend eine Polynukleotid-Bibliothek für die molekulare Charakterisierung der idiopathischen pulmonaren Fibrose, wobei die Bibliothek einen Pool von Polynucleotid-Sequenzen oder Subsequenzen umfasst, worin genannte Sequenzen oder Subsequenzen entweder unterexprimiert oder überexprimiert sind in idiopathisch pulmonaren Fibrosezellen verglichen mit anderen Lungenerkrankungen, wobei die überexprimierten Sequenzen oder Subsequenzen mucin 4, tracheobronchial, AJ242547.1; mucin 5, subtype B, tracheobronchial, A1697108; myosin regulatory light chain 2, smooth muscle isoform, J02854.1; insulin-like growth factor binding protein 2 (36kD), BC004312.1; cadherin 1, type 1, E-cadherin (epithelial), L08599.1; connective tissue growth factor, M92934.1; keratin 15, BC002641.1; cytokeratin 17, Z19574; matrix metalloproteinase 7 (matrilysin, uterine), BC003635.1; cytokeratin 4: X07695.1; insulin-like growth factor 1 receptor: NM_000875.2; keratin 5 (epidermolysis bullosa simplex, Dowling-MearaKobnerWeber-Cockayne types): M21389.1; transforming growth factor b₁, NM_000660 sind, und die unterexprimierten Sequenzen oder Subsequenzen surfactant, pulmonary-associated protein C, BC005913.1; interferon, gamma-inducible protein 30, AF097362.1; interleukin 7 receptor, M29696.1; pulmonary surfactant protein (SP5), J03553; surfactant, pulmonary-associated protein A2, NM_006926.1; interleukin 13 receptor, alpha 2: U70981.1; interferon-gamma-inducible indoleamine 2,3-dioxygenase (IDO): M34455.1; relaxin 1 (H1): BC005956.1. sind,
für die Behandlung von idiopathischer pulmonarer Fibrose, wobei das interferon gamma nach molekularer Charakterisierung der idiopathischen pulmonaren Fibrose, sowie nach erfolgreicher Vorbehandlung der zugrunde liegenden Infektionen angewendet wird.

9. Kit gemäss Anspruch 8, ebenfalls umfassend eine Glucocorticoid-Verbindung.

10. Kit gemäss Anspruch 8, wobei das interferon gamma pegyliertes interferon gamma ist.

11. Kit gemäss Anspruch 10, ebenfalls umfassend eine Glucocorticoid-Verbindung.

12. Kit gemäss Anspruch 8, wobei das interferon gamma in einzelner variablen Dosis von 1,0 bis 5,0 microg / kg Körpergewicht verabreicht wird.

13. Kit gemäss Anspruch 9, wobei die Glucocorticoid-Verbindung in einzelner variablen Dosis von 100 bis 350 microg / kg Körpergewicht verabreicht wird.

14. Kit gemäss Anspruch 8, wobei das interferon gamma zur inhalativen Verabreichung geeignet ist.

## Revendications

1. Utilisation du kit pharmaceutique pour la fabrication d'un médicament pour le traitement de la fibrose pulmonaire idiopathique comprenant
(i) un emballage comprenant l'interféron gamma, éventuellement conjointement avec un support, diluant ou excipient
(ii) un emballage comprenant une bibliothèque polynucléotide pharmaceutique acceptable pour la caractérisation moléculaire de la fibrose pulmonaire idiopathique, ladite bibliothèque comprenant une réserve de séquences ou sous-séquences polynucléotidiques de celui-ci, dans laquelle lesdites séquences ou sous-séquences sont des sous-exprimés ou surexprimés dans les cellules de la fibrose pulmonaire idiopathique, dans lequel séquences ou sous-séquences surexprimés sont mucin 4, tracheobronchial, AJ242547.1; mucin 5, subtype B, tracheobronchial, A1697108; myosin regulatory light chain 2, smooth muscle isoform, J02854.1; insulin-like growth factor binding protein 2 (36kD), BC004312.1; cadherin 1, type 1, E-cadherin (epithelial), L08599.1; connective tissue growth factor, M92934.1; keratin 15, BC002641.1; cytokeratin 17, Z19574; matrix metalloproteinase 7 (matrilysin, uterine), BC003635.1; cytokeratin 4: X07695.1; insulin-like growth factor 1 receptor: NM_000875.2; keratin 5 (epidermolysis bullosa simplex, Dowling-MearaKobnerWeber-Cockayne types): M21389.1; transforming growth factor b₁, NM_000660 et les séquences ou sous-séquences sous-exprimés sont surfactant, pulmonary-associated protein C, BC005913.1; interferon, gamma-inducible protein 30, AF097362.1; interleukin 7 receptor, M29696.1; pulmonary surfactant protein (SP5), J03553; surfactant, pulmonary-associated protein A2, NM_006926.1; interleukin 13 receptor, alpha 2: U70981.1; interferon-gamma-inducible indoleamine 2,3-dioxygenase (IDO): M34455.1; relaxin 1 (H1): BC005956.1.
dans laquelle l'interféron gamma est utilisé après la caractérisation moléculaire de la fibrose pulmonaire idiopathique et après le pré-traitement réussi des infections sous-jacentes.

2. Utilisation du l'interféron gamma selon la revendication 1, comprenant en outré composé glucocorticoïde.

3. Utilisation selon la revendication 1, dans laquelle l'interféron gamma est pégylé interféron gamma.

4. Utilisation du pégylé interféron gamma selon la revendication 3, comprenant en outré composé glucocorticoïde.

5. Utilisation du kit pharmaceutique selon l'une quelconque des revendications précédentes dans laquelle l'interféron gamma est administré à l'individu en une quantité de dose unique allant de 1.0 à 5.0 microg / kg de poids corporel.

6. Utilisation du kit pharmaceutique selon la revendication 2, dans laquelle le composé de glucocorticoïde est administré à l'individu en une quantité de dose unique allant de 100 à 350 microg / kg de poids corporel.

7. Utilisation du kit pharmaceutique selon la revendication 1, dans laquelle l'interféron gamma est administré à l'individu par inhalation.

8. Le kit pharmaceutique comprenant
(i) un emballage comprenant l'interféron gamma, éventuellement conjointement avec un support, diluant ou excipient
(ii) un emballage comprenant une bibliothèque polynucléotide pharmaceutique acceptable pour la caractérisation moléculaire de la fibrose pulmonaire idiopathique, ladite bibliothèque comprenant une réserve de séquences ou sous-séquences polynucléotidiques de celui-ci, dans laquelle lesdites séquences ou sous-séquences sont des sous-exprimés ou surexprimés dans les cellules de la fibrose pulmonaire idiopathique, dans lequel séquences ou sous-séquences surexprimés sont mucin4, tracheobronchial, AJ242547.1; mucin 5, subtype B, tracheobronchial, A1697108; myosin regulatory light chain 2, smooth muscle isoform, J02854.1; insulin-like growth factor binding protein 2 (36kD), BC004312.1; cadherin 1, type 1, E-cadherin (epithelial), L08599.1; connective tissue growth factor, M92934.1; keratin 15, BC002641.1; cytokeratin 17, Z19574; matrix metalloproteinase 7 (matrilysin, uterine), BC003635.1; cytokeratin 4: X07695.1; insulin-like growth factor 1 receptor: NM_000875.2; keratin 5 (epidermolysis bullosa simplex, Dowling-MearaKobnerWeber-Cockayne types): M21389.1; transforming growth factor b₁, NM_000660 et les séquences ou sous-séquences sous-exprimés sont surfactant, pulmonary-associated protein C, BC005913.1; interferon, gamma-inducible protein 30, AF097362.1; interleukin 7 receptor, M29696.1; pulmonary surfactant protein (SP5), J03553; surfactant, pulmonary-associated protein A2, NM_006926.1; interleukin 13 receptor, alpha 2: U70981.1; interferon-gamma-inducible indoleamine 2,3-dioxygenase (IDO): M34455.1; relaxin 1 (H1): BC005956.1. pour le traitement de la fibrose pulmonaire idiopathique, dans laquelle l'interféron gamma est utilisé après la caractérisation moléculaire de la fibrose pulmonaire idiopathique et après le pré-traitement réussi des infections sous-jacentes.

9. Le kit selon la revendication 8, aussi comprenant en outré composé glucocorticoïde.

10. Le kit selon la revendication 8, dans laquelle l'interféron gamma est pégylé interféron gamma.

11. Le kit selon la revendication 10, aussi comprenant en outré composé glucocorticoïde.

12. Le kit selon la revendication 8, dans laquelle l'interféron gamma est administré à l'individu en une quantité de dose unique allant de 1.0 à 5.0 microg / kg de poids corporel.

13. Le kit selon la revendication 9, dans laquelle le composé de glucocorticoïde est administré à l'individu en une quantité de dose unique allant de 100 à 350 microg / kg de poids corporel.

14. Le kit selon la revendication 8, dans laquelle l'interféron gamma est appropriée pour une administration par inhalation.
